(19) Europäisches Patentamt
European Patent Office
Office européen des brevets

(11) Publication number: 0 245 012 B1

(12) **EUROPEAN PATENT SPECIFICATION**

(45) Date of publication of patent specification: **06.02.91**

(51) Int. Cl.$^5$: **C 07 H 17/08,** A 61 K 31/70, C 12 P 19/62 // (C12P19/62, C12R1:785)

(21) Application number: **87303799.8**

(22) Date of filing: **29.04.87**

(54) **Method for the preparation of 14-hydroxy-6-0-methyl-erythromycin A.**

(30) Priority: **02.05.86 JP 102881/86**

(43) Date of publication of application:
**11.11.87 Bulletin 87/46**

(45) Publication of the grant of the patent:
**06.02.91 Bulletin 91/06**

(84) Designated Contracting States:
**AT BE CH DE ES FR GB GR IT LI LU NL SE**

(56) References cited:
**EP-A-0 041 355**
**EP-A-0 222 186**

(73) Proprietor: **TAISHO PHARMACEUTICAL CO. LTD**
**24-1 Takata 3-chome Toshima-ku**
**Tokyo 171 (JP)**

(72) Inventor: **Sasaki, Joji**
**57-2-102, Higashiomiya-4-chome**
**Omiya-shi (JP)**
Inventor: **Mizoue, Kazutoshi**
**8-25-107, Buzo-1-chome**
**Urawa-shi (JP)**
Inventor: **Adachi, Takashi**
**1-5-205, Aoba-1-chome**
**Kuki-shi (JP)**
Inventor: **Nagate, Takatoshi**
**1-8, Mochida-4-chome**
**Gyoda-shi (JP)**
Inventor: **Morimoto, Shigeo**
**121-9, Oaza Homuranakanobun**
**Yoshikawamachi**
**Kitakatsushika-gun Saitama-ken (JP)**
Inventor: **Omura, Sadafumi**
**523-7, Haraichi**
**Ageo-shi (JP)**

**EP 0 245 012 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: Paget, Hugh Charles Edward et al
MEWBURN ELLIS & CO. 2/3 Cursitor Street
London EC4A 1BQ (GB)

## Description

The present invention relates to a method of preparing a 14-hydroxyerythromycin derivative, in particular, 14-hydroxy-6-O-methylerythromycin A and salts thereof.

An antibiotic 6-O-methylerythromycin A represented by the formula

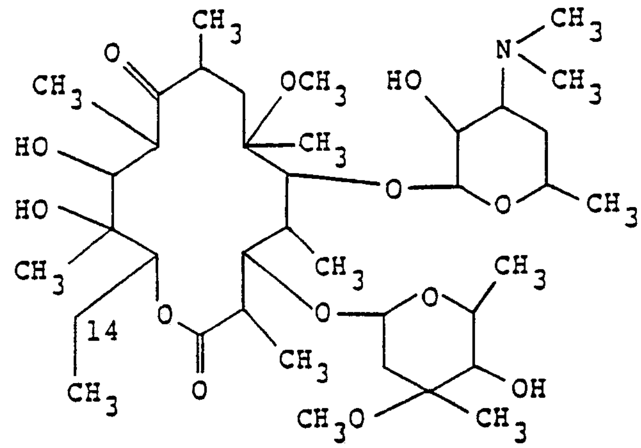

is disclosed in our US—A—4331803. This compound is an excellent antibiotic, but there is a need to find more effective antibiotics.

The compound 14-hydroxy-6-O-methylerythromycin A and its preparation from 6-O-methylerythromycin A by incorporating it into a liver homogenate and incubating the mixture is disclosed in EP—A—0222186, which falls under Art. 54(3) EPC.

As a result of earnest research to meet the need for more effective antibiotics, we found that the above-mentioned compound having a hydroxy group at the 14-position of 6-O-methylerythromycin A can be prepared by reacting 6-O-methylerythromycin A with a specific microorganism, as hereinafter described.

Although it is difficult to prepare this compound by chemical means, it can be easily prepared by using the microorganism. Furthermore, this compound has strong *in vitro* antibacterial activity against Gram-positive bacteria and some Gram-negative bacteria, especially *Neissenia gonorroeae* and *Haemophilus influenzae*, and has stronger *in vivo* antibacterial activity against some Gram-positive bacteria than 6-O-methylerythromycin A.

Thus, the present invention provides a process by which 14-hydroxy-6-O-methylerythromycin A or a salt thereof having excellent antibacterial activity can be easily prepared.

Characteristics of this compound are illustrated by the accompanying drawings in which Fig. 1 shows the IR absorption spectrum of 14-hydroxy-6-O-methylerythromycin A measured in KBr tablet, Fig. 2 shows the $^{1}H$ NMR spectrum of 14-hydroxy-6-O-methylerythromycin A measured in pyridine-d5 at 400 MHz, and Fig. 3 shows the $^{13}C$-NMR spectrum of 14-hydroxy-6-O-methylerythromycin A measured in pyridine-d5 at 100 MHz.

The compound 14-hydroxy-6-O-methylerythromycin A is represented by the formula

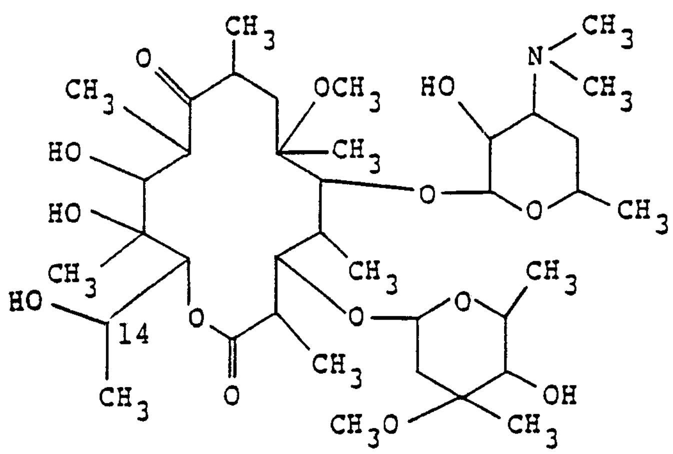

and a method for preparing the compound, or a salt thereof, comprises cultivating *Mucor circinelloides* f. *griseo-cyanus* IFO 4563 in a medium containing 6-O-methylerythromycin A, and optionally salifying the resultant 14-hydroxy-6-O-methylerythromycin A.

The salt may be any pharmaceutically acceptable salt with an organic acid such as tartaric acid, citric acid, acetic acid, stearic acid, succinic acid, methanesulfonic acid, or aminoethanesulfonic acid; or an amino acid such as aspartic acid or glutamic acid.

*Mucor circinelloides* f. *griseo-cyanus* IFO 4563 is a storagae strain in the public culture collection at the Institute for Fermentation, Osaka and its mycological features are already known. It has been found by the research of the present inventors that this strain has an ability to introduce a hydroxy group to the 14-membered macloride compounds at the 14-position. The method of the present invention can be carried out by cultivating *Mucor circinelloides* f. *griseo-cyanus* IFO 4563 in the medium containing 6-O-methylerythromycin A as a substrate under aerobic conditions.

A liquid medium is chiefly used, and the carbon sources used may be saccharose, glucose and dextrose, and they may be used alone or in admixture. Nitrogen sources which may be used are polypeptone, sodium nitrate, yeast extract and the like, and they may be used alone or in admixture. In addition, if necessary, organic and inorganic acids can be added in order to aid the growth of the strain and to promote the production of 14-hydroxy-6-O-methylerythromycin A. Examples of defoaming agents which can be used are Adecanol (produced by Asahi Denka Kogyo Co.) and silicon.

Preferred cultivations are aerobic cultivations such as shake cultivation and aerobic stirring cultivation at pH 6 to 7 at a temperature of 28 to 30°C for 4 to 8 days.

Furthermore, a suitable amount of 6-O-methylerythromycin A is preferably added at an early stage of the cultivation.

Isolation of 14-hydroxy-6-O-methylerythromycin obtained by the cultivation can be carried out by the common procedures for recovering the fermentation product. Namely, after completion of the cultivation, the culture solution isolated by centrifugation or filtration is adsorbed on a carrier such as Diaion HP 20 (trade name of Mitsubishi Chemical Industrial Co.) and eluted with a solvent such as a lower alcohol or acetone. The eluted fractions containing 14-hydroxy-6-O-methylerythromycin A are concentrated and then crystallized from ethanol to give the crude crystals of 14-hydroxy-6-O-methylerythromycin A, which are then dissolved in a mixture of chloroform, methanol and aqueous ammonia. The solution is subjected to silica gel column chromatography (e.g., Kieselgel 60, produced by Merck, West Germany) followed by gel filtration on Sephadex LH-20 (trade name of Pharmacia Fine Chemical Co.) with methanol or ethanol as an eluent to isolate 14-hydroxy-6-O-methylerythromycin A.

The physicochemical properties of 14-hydroxy-6-O-methylerythromycin A obtained by the method of the present invention are as follows:

Physiochemical Properties

(1) Appearance: white needles;

(2) Melting point: 214.5—216.5°C;

(3) Elemental analysis:

| Found | Calculated |
|---|---|
| C: 59.27% | C: 59.74% |
| H: 9.12% | H: 9.10% |
| N: 1.95% | N: 1.83% |

(4) Molecular formula: FD and EI mas spectra

FD: $(M + H)^+$ m/z 764

EI: $(M)^+$ m/z 763

(5) Molecular formula: $C_{38}H_{69}NO_{14}$

(6) UV absorption: 280 nm (ε 50) in an ethanol solution;

(7) IR absorption spectrum: A spectrum obtained in KBr tablet is shown in Fig. 1.

(8) $^1$H-NMR spectrum: A spectrum obtained in pyridine-d5 at 400 MHz is shown in Fig. 2.

(9) $^{13}$C-NMR spectrum: A spectrum obtained in pyridine-d5 at 100 MHz is shown in Fig. 3.

(10) Solubility:

Soluble in chloroform, methanol, ethanol, acetone, ethyl acetate and pyridine.

Poorly soluble in ethyl ether, n-hexane, petroleum ether, benzene and water.

(11) Color reaction:

Positive with sulfuric acid, iodide, anisaldehyde-sulfuric acid and vaniline-sulfuric acid.

Negative with an aqueous ferric chloride solution and ninhydrin.

(12) Nature: Basic.

14-hydroxy-6-O-methylerythromycin A and the salts thereof prepared by the method of the present invention have strong *in vitro* antibacterial activity against Gram-positive bacteria and some Gram-negative bacteria, especially *Neissenia gonorrhoeae* and *Haemophilus influenzae*, and have stronger *in vivo* antibacterial activity against some Gram-positive bacteria than 6-O-methylerythromycin A, and therefore, they can be used as antibiotics. The effective amount ($ED_{50}$) of 14-hydroxy-6-O-methylerythromycin A is from 3 mg/kg to 10 mg/kg of body weight. For the purpose, the compound prepared by the method of the present invention may be administered orally or parenterally in a conventional dosage form such as tablet, capsule, powder, troche, ointment, suspension or solution prepared according to conventional

pharmaceutical practices. The $LD_{50}$ value of the compound in ICR mice is 560 mg/kg of body weight by intravenous administration.

As stated above, using the method of the present invention, it is easy to introduce a hydroxy group into the 14-membered macloride compounds at the 14-position which had not previously been achieved by chemical means so that 14-hydroxy-6-O-methylerythromycin A and the salts thereof can be prepared easily and effectively.

A method embodying the present invention is illustrated in more detail by the following Example and Experiments.

## Example

(1) *Mucor circinelloides* f. *griseo-cyanus* IFO 4563 was inoculated into a sterile liquid medium (pH 7.0) containing 1% of sacchrose, 0.3% of peptone, 0.3% of yeast extract and 50 μg/ml of 6-O-methylerythromycin A, and shake cultivation was carried out at 30°C for 48 hours to give a seed culture. Then, 30 ml of the seed culture was inoculated into 3 l of a sterile liquid medium (pH 7.0) containing 5% of saccharose, 0.2% of peptone, 0.1% of a yeast extract, 0.1% of dipotassium hydrogen phosphate, 0.05% of potassium chloride, 0.05% of magnesium sulfate and 80 μg/ml of 6-O-methylerythromycin A using a 5 l fermentation jar, and fermentation was carried out under aerobic conditions at 30°C for 192 hours. The same fermentation was carried out in a total of 15 l of the medium using 5-fermentation jars.

(2) After completion of the fermentation, the culture solution was filtered to separate the mycelium and the filtrate. The filtrate thus obtained was adsorbed on 1.5 l of Dialon HP-20 (trade name, Mitsubishi Chemical Co.) and eluted with 3 l of ethanol. The elutate was concentrated to give crude crystals of a mixture of 14-hydroxy-6-O-methylerythromycin A and 6-O-methylerythromycin A. The crude crystals were dissolved in a mixture of chloroform, methanol and 25% aqueous ammonia (20:1:0.1 v/v) and adsorbed on a silica gel (Kieselgel 60: trade name of Merck) column packed with the same mixture as that described above. The silica gel column was eluted with 1.2 l of the same mixture as that described above, and the fractions containing 14-hydroxy-6-O-methylerythromycin A were obtained which were confirmed by silica gel thin layer chromatography (Kieselgel 60 F254: trade name of Merck). These fractions were concentrated to dryness to obtain 120 mg of crude 14-hydroxy-6-O-methylerythromycin A as powder.

(3) The crude product obtained in the above item (2) was dissolved in 6 ml of methanol, the solution was subjected to gel filtration on a column of 250 ml of Sephadex LH-20 (trade name of Pharmacia Fine Chemicals) with methanol as an eluent, and the fractions containing 14-hydroxy-6-O-methylerythromycin A were obtained which were confirmed by thin layer chromatography. These fractions were collected and concentrated to dryness and recrystallized from a little amount of ethanol to give about 100 mg of 14-hydroxy-6-O-methylerythromycin A as white needles, m.p. 214.5—216.5°C.

Experiment 1

(*In vitro* antibacterial activity)

The antibacterial activity (MIC: minimum inhibitory concentration) of 14-hydroxy-6-O-methylerythromycin A was measured according to the MIC method specified by the Japan Chemotherapeutic Society. The results are shown in Table 1.

TABLE 1

| Test microorganis | MIC (μg/ml) |
|---|---|
| *Staphylococcus aureus* 209P-JC | 0.10 |
| *Staphylococcus aureus* BB | 0.20 |
| *Staphylococcus aureus* Smith 4 | 0.20 |
| *Staphylococcus epidermis* IID 866 | 0.20 |
| *Staphylococcus pneumoniae* IDD 552 | 0.025 |
| *Staphylococcus pneumoniae* IDD 553 | 0.025 |
| *Staphylococcus pyogenes* IID 689 | 0.025 |
| *Bacillus subtilis* ATCC 6633 | 0.10 |
| *Micrococcus luteus* NIHJ | 0.05 |
| *Micrococcus luteus* ATCC 9341 | 0.025 |
| *Enterococcus faecalis* ATCC 8043 | 0.05 |
| *Escherichia coli* NIHJ JC-2 | 50 |
| *Klebsiella pneumoniae* IFO 3317 | 25 |
| *Salmonella enteritidis* | 25 |
| *Branhamella catarrhalis* | 0.05 |
| *Neisseria gonorrhoeae* TCC-4 | 0.39 |
| *Haemophilus influenzae* J-48 | 3.13 |

Experiment 2

(*In vivo* antibacterial test)

The *in vivo* antibacterial activity of 14-hydroxy-6-O-methylerythromycin A was measured by effect on treatment of experimental infectious diseases of mouse. 6-O-methylerythromycin A and erythromycin A were used as comparative drugs. *Staphylococcus aureus* BB and *Streptococcus pneumoniae* IID 553 were used as experimental infectious diseases of mouse. Ten male ICR mice, weighing 26 ± 1 g, were used for each group to a different concentration of one drug. The bacteria were administered intraperitoneally to each groups of rats for infection. 14-hydroxy-6-O-methylerythromycin A, 6-O-methylerythromycin A and erythromycin A were administered orally to different groups of mice. After observing for 7 days, $ED_{50}$ value was calculated from the number of mice surviving.

The $ED_{50}$ values having 95% confidence limits were estimated by probit analysis. The test results are shown in Table 2.

TABLE 2

| Drug \ Bacteria | *Staphylococcus aureus* BB $ED_{50}$ (mg/mouse) | *Streptococcus pneumoniae* IID 553 $ED_{50}$ (mg/mouse) |
|---|---|---|
| 14-Hydroxy-6-O methylerythromycin A | 0.234 | 0.069 |
| 6-O-Methylerythromycin A | 0.394 | 0.222 |
| Erythromycin A | 2.20 | 1.43 |

**Claim**

A method for preparing 14-hydroxy-6-O-methylerythromycin A represented by the formula

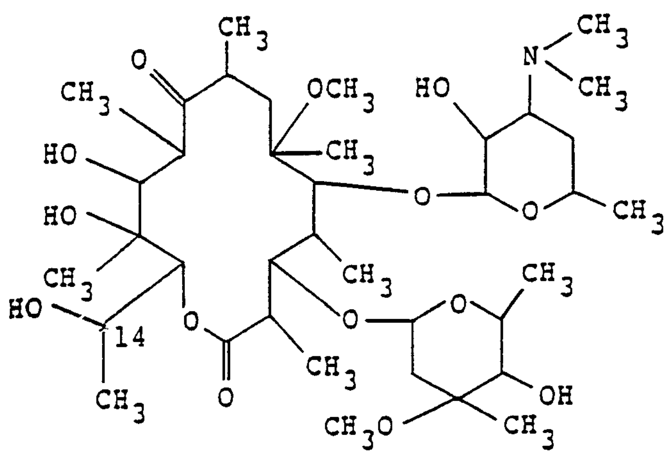

or a salt thereof, which method comprises cultivating *Mucor circinelloides* f. *griseo-cyanus* IFO 4563 in a medium containing 6-O-methylerythromycin A, and optionally salifying the resultant said 14-hydroxy-6-O-methylerythromycin A.

**Patentanspruch**

Verfahren zur Herstellung von 14-Hydroxy-6-O-methylerythromycin A der Formel

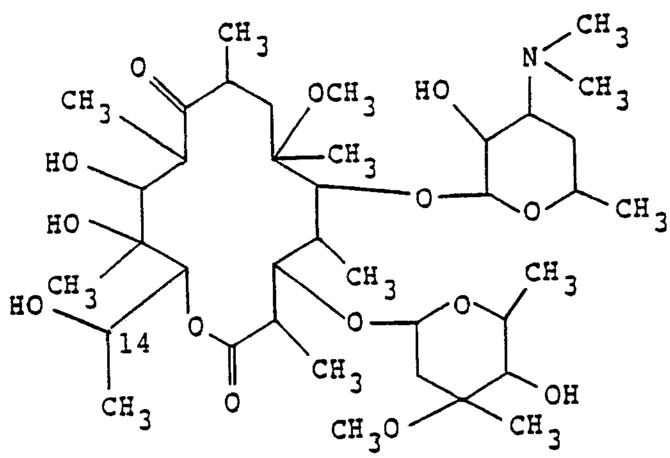

oder eines Salzes davon, welches Verfahren das Kultivieren von Mucor circinelloides f. griseo-cyanus IFO 4563 in einem 6-O-Methylerythromycin A enthaltenden Medium und gegebenenfalls eine Salzbildung des resultierenden 14-Hydroxy-6-O-methylerythromycin A umfaßt.

**Revendication**

Procédé de préparation de la 14-hydroxy-6-O-méthylérythromycine A répondant à la formule:

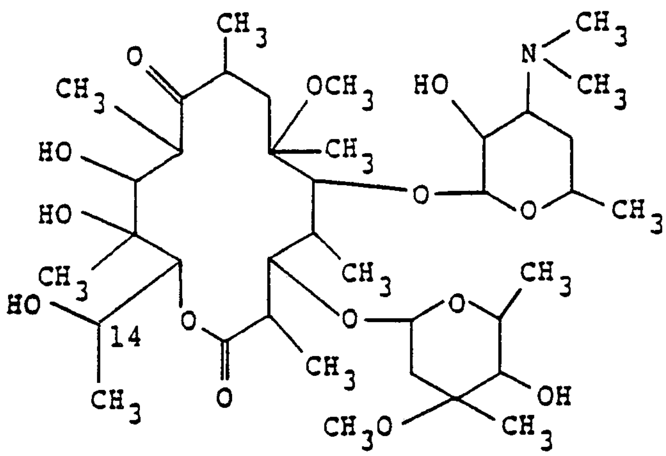

ou de ses sels, lequel procédé comprend le fait de cultiver Mucor circinelloides f. griseo-cyanus IFO 4563 dans un milieu contenant de la 6-O-méthylérythromycine A, et si on le désire, le fait de salifier la 14-hydroxy-6-O-méthylérythromycine A obtenue.

FIG. 1

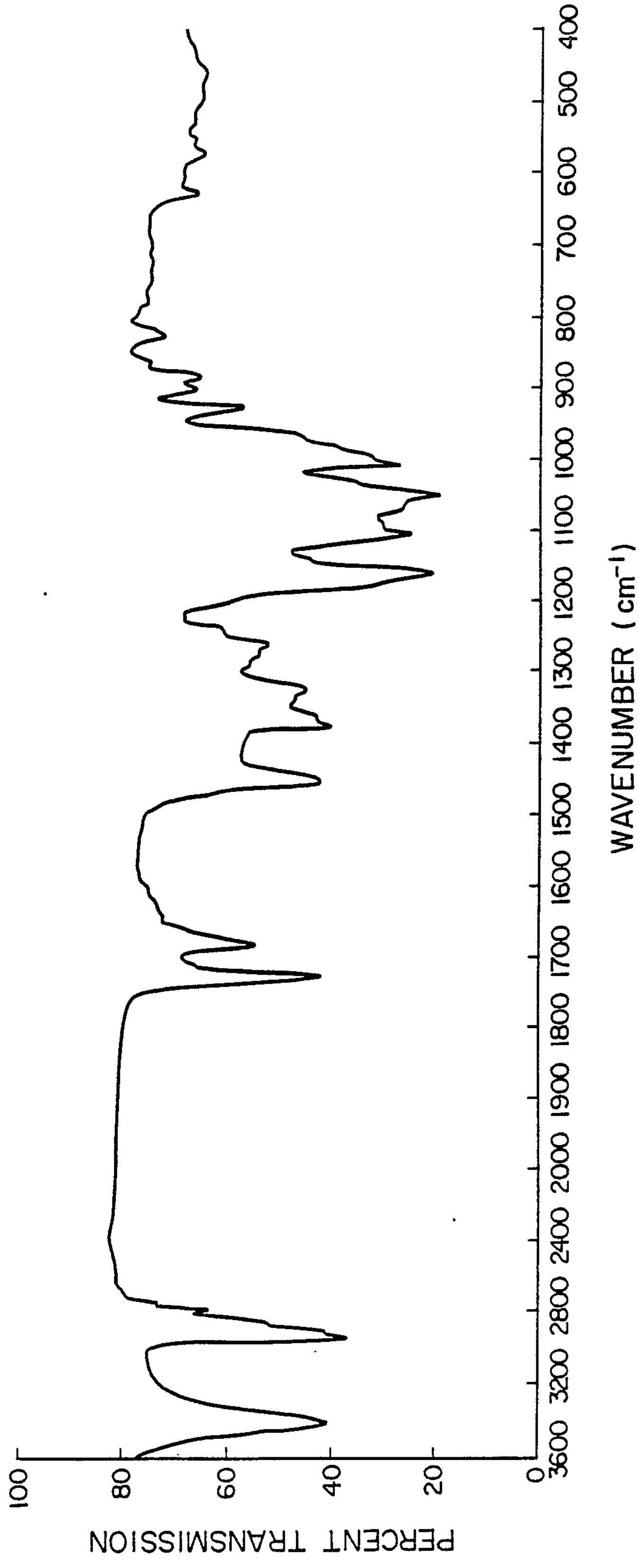
PERCENT TRANSMISSION
100
80
60
40
20
0
WAVENUMBER (cm$^{-1}$)
3600 3200 2800 2400 2000 1900 1800 1700 1600 1500 1400 1300 1200 1100 1000 900 800 700 600 500 400

FIG. 2

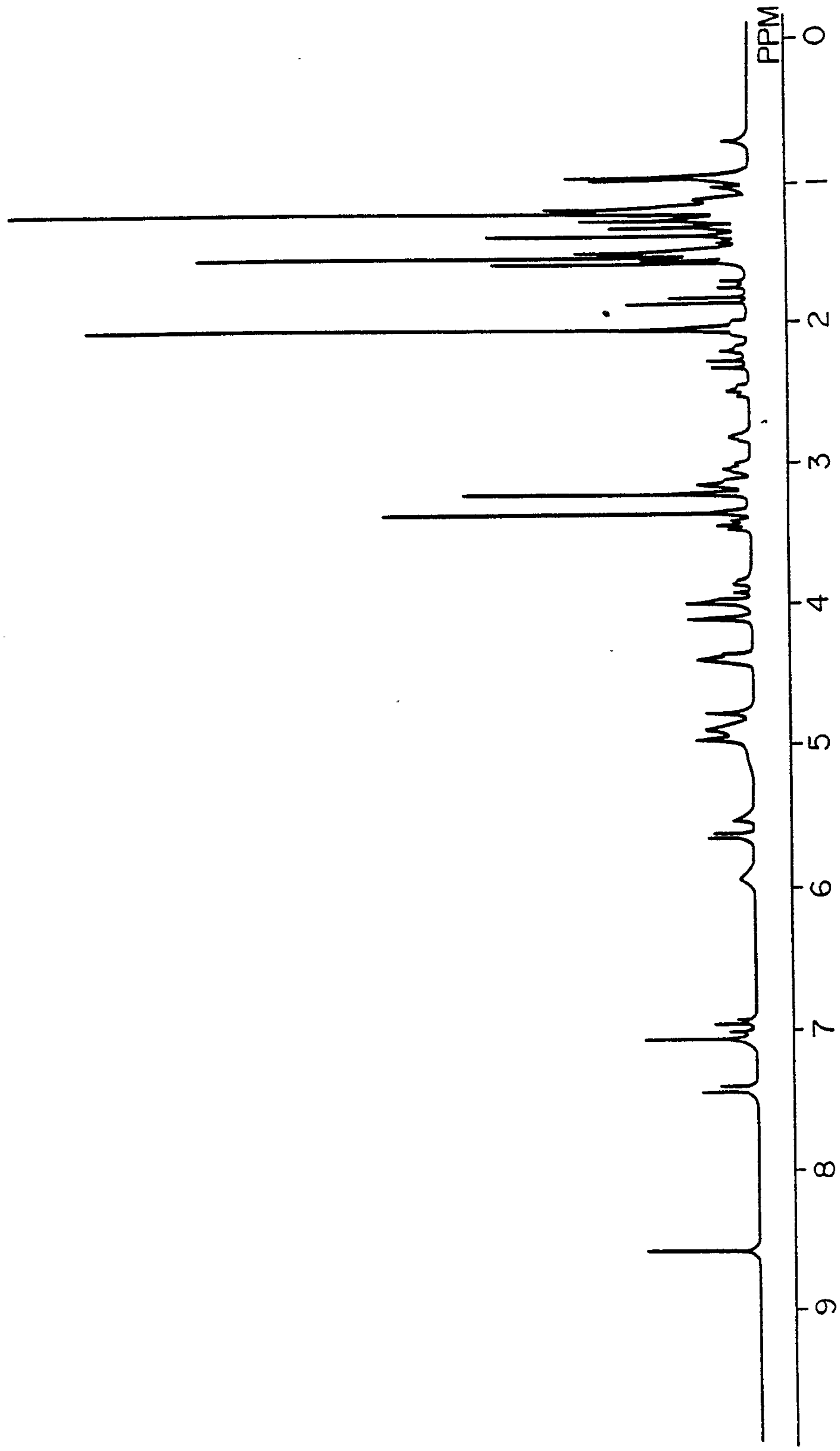

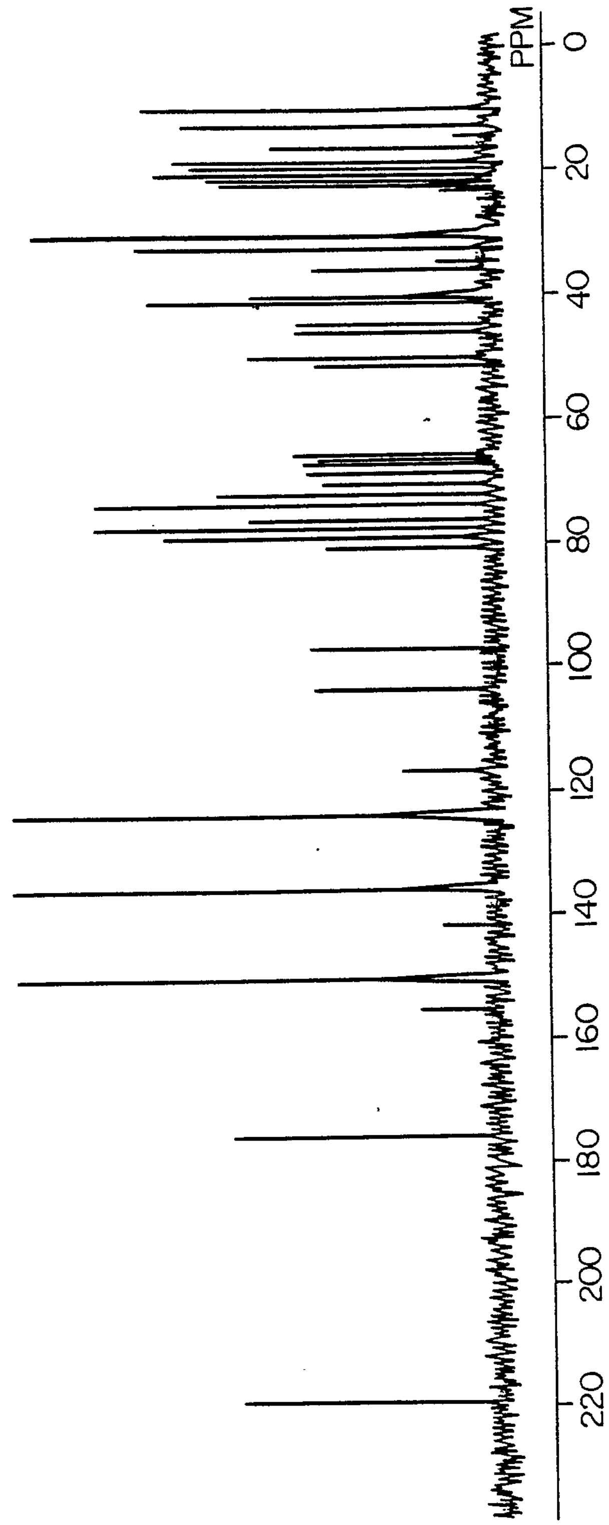


FIG. 3